# FASCICULE DE BREVET EUROPEEN

(11) **EP 2 729 495 B1**
(45) Date de publication et mention de la délivrance du brevet: **17.08.2016**
(21) Numéro de dépôt: 12738574.8
(22) Date de dépôt: 06.07.2012
(51) Int. Cl.: C07K 14/78, C07K 7/06, C07K 7/08, C07K 7/64, A61K 38/00

(54) **PEPTIDE ANTAGONISTE DE LA LIAISON ENTRE LE CD47 ET UNE PROTEINE APPARTENANT A LA FAMILLE DES THROMBOSPONDINES**
PEPTIDISCHER ANTAGONST DER BINDUNG ZWISCHEN CD47 UND EINEM DER FAMILIE DES THROMBOSPONDINS GEHÖRENDEN PROTEIN
PEPTIDIC ANTAGONIST OF THE BINDING BETWEEN CD47 AND A PROTEIN BELONGING TO THE FAMILY OF THROMBOSPONDIN

(30) Priorité: 08.07.2011 FR 1156237
(43) Date de publication de la demande: 14.05.2014
(73) Titulaire: Université De Reims Champagne-Ardenne, 51100 Reims (FR)
(72) Inventeur: DEDIEU, Stéphane, F-51100 Reims (FR); FLOQUET, Nicolas, F-34725 Saint Andre de Sangonis (FR); MARTINY, Laurent, F-51100 Reims (FR); SCHNEIDER, Christophe, F-51170 Aubilly (FR); JEANNE, Albin, F-51100 Reims (FR); SICK, Emilie, 51100 Reims (FR); DAUCHEZ, Manuel, F-51390 Rosnay (FR)
(74) Mandataire: Rhein, Alain
(86) Numéro de dépôt international: PCT/FR2012/051593
(87) Numéro de publication internationale: WO 2013/007933

(56) Documents cités:
- WO-A1-97/27873
- WO-A2-2010/017332

### LISTAGE DE SEQUENCES

<110> Université de Reims Champagne Ardenne
<120> Peptide antagoniste de la liaison entre le CD47 et une protéine appartenant à la famille des thrombospondines
<130> 9U11 BT PCT 1
<150> FR 1156237 <151> 2011-07-08
<160> 2
<210> 1
   <211> 12
   <212> PRT
   <213> Séquence artificielle
<220>
   <223> Peptide antagoniste de la liaison entre un récepteur CD47 et une protéine appartenant à la famille des thrombospondines
<400> 1
   Ile Glu Val Ser Gln Leu Leu Lys Gly Asp Ala Ser
    1               5                   10
<210> 2
   <211> 12
   <212> PRT
   <213> Séquence artificielle
<220>
   <223> Peptide antagoniste de la liaison entre un récepteur CD47 et une protéine appartenant à la famille des thrombospondines
<400> 2
   Cys Glu Val Ser Gln Leu Leu Lys Gly Asp Ala Cys
    1               5                   10

## Description

La présente invention concerne le domaine des interactions moléculaires entre deux protéines.

L'invention concerne plus particulièrement les interactions entre une protéine extracellulaire appartenant à la famille des thrombospondines et un récepteur, le CD47, situé à la surface de la membrane cellulaire.

La présente invention trouvera une application potentielle principalement dans le domaine des pathologies tumorales, thrombotiques et cardiovasculaires.

L'invention concerne plus particulièrement un peptide ayant la capacité de lier spécifiquement le domaine de liaison au récepteur CD47 d'une protéine appartenant à la famille des thrombospondines, de manière à empêcher la liaison entre les deux protagonistes.

Les thrombospondines (TSP), et en particulier la TSP-1 et la TSP-2, sont des protéines connues pour avoir la capacité de se lier au récepteur CD47 ce qui entraine un signal aboutissant à une réponse cellulaire de la part de la cellule. Une telle interaction semble jouer un rôle important notamment dans des processus cellulaire fondamentaux, tels que la régulation de la mort cellulaire programmée, également appelée apoptose, ou l'inflammation.

Par exemple, des travaux récemment réalisés par les inventeurs ont permis de mettre en évidence le rôle anti-apoptotique de la TSP-1 sur des cellules cancéreuses de carcinome folliculaire thyroïdien humain, également appelées cellules FTC (Rath et al., 2006). D'autres études ont également démontré qu'une diminution de l'expression de la TSP-1 entraine la réversibilité du phénotype des carcinomes squameux grâce à l'utilisation d'une stratégie antisens.

Des recherches supplémentaires, menées pour essayer de mettre en évidence le rôle de la TSP-1 dans le développement des cellules du cancer du sein, ont quant à elles apporté des résultats contradictoires ; en effet, certaines recherches concluent que la TSP-1 présente un effet pro-apoptotique, c'est-à-dire qu'elle inhiberait la croissance de la tumeur mammaire (Esemuede et al., 2004 ; Manna et Frazier 2004). Au contraire, d'autres études défendent le concept inverse selon lequel la TSP-1 jouerait un rôle anti-apoptotique et/ou augmenterait les propriétés invasives de ces cellules tumorales (Wang et al., 1996a, 1996b).

La compréhension des mécanismes moléculaires aboutissant à l'interaction entre les protéines appartenant à la famille des thrombospondines et leurs récepteurs, ainsi que la découverte des réponses cellulaires en découlant, constituent donc une étape importante pour mettre en place des stratégies thérapeutiques destinées à inhiber le développement et la prolifération des cellules cancéreuses.

Certains documents de l'état de l'art préconisent déjà d'utiliser les propriétés d'interaction entre les thrombospondines et leurs récepteurs, en particulier le CD47, afin de développer des stratégies pour soit diminuer, soit augmenter, le taux d'apoptose des cellules que celles-ci soient cancéreuses ou non.

Ainsi, il est connu de l'état de la technique, par exemple du document de brevet US 7,582,725 B2 d'utiliser un agent se liant soit au récepteur CD47, soit à la thrombospondine-1 de manière à inhiber la liaison entre ladite TSP-1 et ledit récepteur CD47. Le fait d'empêcher cette interaction permettrait de réduire significativement le taux d'apoptose de certaines cellules, telles que les fibroblastes ou les cellules épithéliales, impliquées notamment dans les processus de cicatrisation dans lesquels elles jouent un rôle important. Préférentiellement, dans le but de diminuer le taux d'apoptose induit par la protéine TSP-1, un peptide présentant une séquence amino-acidique de formule générale R-A1-Y-V-V-M est utilisé.

Cependant, dans le cas de la présente invention, les cellules à cibler et le but à atteindre sont complètement différents. En effet, il ne s'agit pas dans notre cas d'essayer d'enrayer l'apoptose cellulaire mais au contraire de favoriser la mort des cellules cancéreuses (apoptose et/ou nécrose). Ainsi, le peptide proposé par le document susmentionné n'est pas adapté pour résoudre le problème posé, à savoir inhiber le potentiel invasif des cellules cancéreuses, en particulier en favorisant l'entrée en apoptose de ces dernières.

On connait également dans l'état de la technique, l'utilisation d'anticorps monoclonaux dirigés spécifiquement soit contre la protéine TSP, soit contre le récepteur CD47. Cela a pour conséquence d'empêcher l'interaction entre ces deux protéines, et donc d'inhiber les réponses cellulaires qui en découlent.

On connait ainsi le document WO 97/27873 qui décrit des anticorps monoclonaux qui lient spécifiquement le CD47. Cependant, l'inconvénient principal de cette technique est que les anticorps monoclonaux dirigés contre l'une ou l'autre des protéines ne vont pas se lier spécifiquement au niveau précis du site de liaison entre la TSP et le CD47. Ainsi, le blocage de l'interaction entre les deux protéines peut ne pas être optimal et la fixation de l'anticorps, que ce soit sur la TSP ou sur le CD47, peut empêcher l'interaction de ces protéines avec leurs autres ligands naturels. En conséquence, d'autres processus cellulaires, qui sont indispensables à l'organisme et qui mettent en oeuvre l'une ou l'autre des protéines susmentionnées, peuvent ainsi être inhibés ou abolis. De plus, un autre inconvénient majeur d'une telle stratégie réside dans le fait que l'anticorps anti-CD47 peut agir dans certains cas comme un agoniste du récepteur, c'est-à-dire qu'il peut activer celui-ci par l'intermédiaire de l'interaction.
Le document de brevet CA 244 6391 utilise également les propriétés de liaison entre le récepteur CD47 et son ligand, la protéine TSP-1. Plus particulièrement, il s'agit du rôle de ces deux molécules dans la réaction immunitaire, et notamment dans la réaction inflammatoire, qui est exploité. Le document divulgue ainsi l'utilisation d'un anticorps monoclonal dirigé contre le récepteur CD47 afin notamment d'inhiber l'activité des lymphocytes T suppresseurs. Une telle inhibition jouerait un rôle bénéfique dans des processus cellulaires variés, en particulier dans la neutralisation d'agents infectieux, les réactions allergiques, les maladies auto-immunes ou inflammatoires, etc.

Cependant, comme expliqué ci-dessus, l'utilisation d'un anticorps monoclonal dirigé contre l'une ou l'autre des deux protéines ne constitue pas une solution satisfaisante.

Le document WO 2010/017332 A2 est également relatif à l'inhibition de l'interaction entre le récepteur CD47 et la thrombospondine. En particulier, ce document présente l'utilisation d'agents très divers pour inhiber cette interaction préalablement au traitement par radiothérapie d'un patient, de manière à faciliter l'ablation de la tumeur. En effet, empêcher l'interaction TSP-1/CD47 favoriserait la protection des cellules du système immunitaire contre les dommages provoqués par une exposition de celles-ci aux radiations. De ce fait, la réponse immunitaire à l'encontre des cellules tumorales serait considérablement augmentée.

Cependant, les agents utilisés ne sont pas adaptés à bloquer spécifiquement le site de liaison de la TSP au récepteur CD47. L'invention offre la possibilité de pallier, au moins en partie, les divers inconvénients de l'état de la technique en proposant un peptide antagoniste de l'interaction du CD47 avec des protéines appartenant à la famille des thrombospondines, en particulier les TSP-1 et 2. De façon particulièrement avantageuse, le peptide se fixe uniquement au niveau du domaine de la TSP qui interagit avec le récepteur CD47, de manière à laisser libre les autres domaines de la TSP et l'intégralité du domaine extracellulaire du CD47 pour qu'ils puissent lier leurs ligands naturels. La nature de l'invention, c'est-à-dire le peptide antagoniste, permettrait de faciliter la délivrance de la substance active, et d'améliorer sa biodisponibilité au niveau des cellules cibles tout en limitant le caractère immunogène. Un autre avantage réside dans le fait que le peptide antagoniste permet à la fois d'inhiber la liaison du CD47 avec la TSP-1 et avec la TSP-2.

A cet effet, la présente invention concerne un peptide antagoniste de la liaison entre un récepteur CD47, et une protéine appartenant à la famille des thrombospondines, ou TSP.

Le peptide selon l'invention est particulier en ce qu'il présente la séquence S1 suivante :
S1: R1-R2-R3-S-Q-L-L-K-G-R4-R5-R6

De façon particulièrement avantageuse, ledit peptide interagit spécifiquement avec l'extrémité C-terminale de la TSP, au niveau du site de liaison entre ladite TSP et ledit récepteur CD47, de manière à empêcher une interaction entre ledit récepteur CD47 et ladite protéine.

Selon un exemple de réalisation, les radicaux R1, R3 et R5 correspondent chacun à un acide aminé apolaire choisi parmi l'isoleucine (I) et/ou la leucine (L) et/ou la valine (V) et/ou l'alanine (A).

Selon un autre exemple de réalisation intéressant, les radicaux R2 et R4 correspondent chacun à un acide aminé polaire chargé négativement choisi parmi l'acide glutamique (E) et/ou l'acide aspartique (D).

De façon avantageuse, R6 correspond à un acide aminé polaire et non chargé comportant un radical hydroxyle choisi parmi la sérine (S) ou la thréonine (T).

Bien évidemment, ces modes de réalisation ne sont pas limitatifs de l'invention. En effet, comme mentionné ci-dessous, les radicaux R1 et R6 peuvent correspondre à la cystéine (C) de manière à permettre une cyclisation du peptide.

De manière particulièrement avantageuse, la séquence S1 du peptide selon l'invention est égale à la séquence suivante identifiée S2:
S2: I-E-V-S-Q-L-L-K-G-D-A-S

Selon un mode de réalisation particulièrement préférentiel, le peptide selon la présente invention, dont la formule est représentée ci-dessus, est cyclisé.

Plus préférentiellement encore, la cyclisation dudit peptide est effectuée par l'intermédiaire d'un pont disulfure entre deux acides aminés de type cystéine (C).

Ainsi, de façon particulièrement avantageuse, le peptide selon la présente invention présente la séquence S1 égale à la séquence suivante, identifiée séquence S3:
S3: C-E-V-S-Q-L-L-K-G-D-A-C

Selon un mode de réalisation avantageux, le peptide selon l'invention est obtenu par voie recombinante.

De façon encore plus préférentielle, ledit peptide est obtenu par synthèse chimique.

L'invention concerne également l'utilisation du peptide dans le cadre du traitement contre le cancer.

Plus particulièrement, le peptide peut être utilisé notamment dans le cadre du traitement contre le cancer folliculaire thyroïdien, le cancer mammaire ou le mélanome.

Cependant, ce listing n'est pas exhaustif et le peptide selon l'invention pourrait également s'avérer intéressant pour le traitement d'autres types de cancer, par exemple le cancer du pancréas ou du colon.

L'invention est également relative à une utilisation du peptide pour empêcher l'interaction entre la thrombospondine-1 et le récepteur CD47 et inhiber l'effet anti-apoptotique de cette interaction sur les cellules cancéreuses.

L'invention concerne encore une utilisation du peptide pour empêcher l'interaction entre la thrombospondine-2 et le récepteur CD47 et inhiber l'effet anti-apoptotique de cette interaction sur les cellules cancéreuses.

La présente invention concerne encore un acide nucléique isolé codant pour le peptide selon la présente invention.

Dans la présente description, le code international à une lettre des acides aminés est utilisé. Ainsi A correspond à l'alanine (Ala), C à la cystéine (Cys), D à l'acide aspartique (Asp), E à l'acide glutamique (Glu), F à la phénylalanine (Phe), G à la glycine (Gly), H à l'histidine (His), I à l'isoleucine (Ile), K à la lysine (Lys), L à la leucine (Leu), M à la méthionine (Met), N à l'asparagine (Asn), P à la proline (Pro), Q à la glutamine (Gln), R à l'arginine (Arg), S à la sérine (Ser), T à la thréonine (Thr), V à la valine (Val), W au tryptophane (Trp) et Y à la tyrosine (Tyr).

La présente invention comporte de nombreux avantages. D'une part, le peptide antagoniste se fixe uniquement au niveau du domaine de la TSP qui interagit avec le récepteur CD47, de manière à laisser libre les autres domaines de la TSP et l'intégralité du domaine extracellulaire du CD47 pour qu'ils puissent lier leurs ligands naturels. Cela permet de faciliter la délivrance de la substance active, ici le peptide antagoniste, et d'améliorer sa biodisponibilité au niveau des cellules cibles tout en limitant le caractère immunogène. D'autre part, le peptide antagoniste permet à la fois d'inhiber la liaison du CD47 avec la TSP-1 et avec la TSP-2. En effet, la majorité des travaux ont été conduits sur la TSP-1 mais celle-ci présente une forte homologie avec la TSP-2.

De plus, ce pourcentage d'homologie entre la TSP-1 et la TSP-2 est particulièrement important au niveau de leur extrémité carboxy-terminale (C-terminale), plus particulièrement encore au niveau de la séquence incriminée, ce qui laisse à penser d'une part que la TSP-2 agit de manière similaire à la TSP-1 vis-à-vis du récepteur CD47 et que d'autre part le peptide selon la présente invention va permettre d'inhiber également l'interaction entre la protéine TSP-2 et le récepteur CD47.

D'autres caractéristiques et avantages de l'invention ressortiront de la description détaillée qui va suivre des modes de réalisation non limitatifs de l'invention, en référence aux figures annexées dans lesquelles :

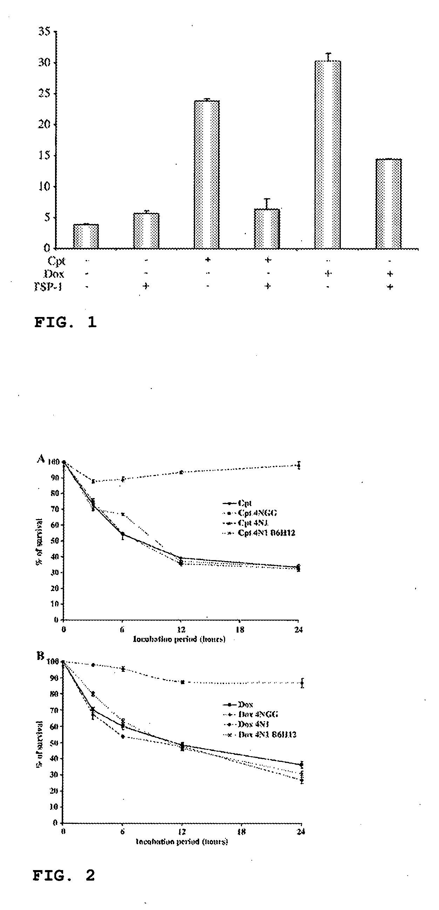

- la figure 1 illustre l'activité caspase-3, marqueur de l'apoptose cellulaire, obtenue en mesurant la quantité de para-nitroaniline libérée par heure et par µg de protéine (en ordonnées). L'activité caspase-3 est mesurée sur des cellules de carcinome folliculaire thyroïdien en présence d'une molécule anticancéreuse induisant l'apoptose, la camptothécine (Cpt) ou la doxorubicine (Dox) en présence ou en l'absence de TSP-1.
- la figure 2 illustre le rôle du peptide 4N1 (K-R-F-Y-V-V-M-W-K), dérivé du domaine C-terminal de la TSP-1, et du récepteur CD47 sur la régulation de l'apoptose induite par la camptothécine (Cpt) ou la doxorubicine (Dox). Pour cela, les cellules de carcinome thyroïdien (FTC-133) ont été incubées en présence de 5 µM de l'une ou l'autre drogue et 100 µM de peptide test 4N1 avec ou sans anticorps B6H12 (100µg/mL) bloquant le récepteur CD47. La même expérience a été conduite avec un peptide témoin 4NGG (K-R-F-Y-G-G-M-W-K), incapable de lier le CD47.

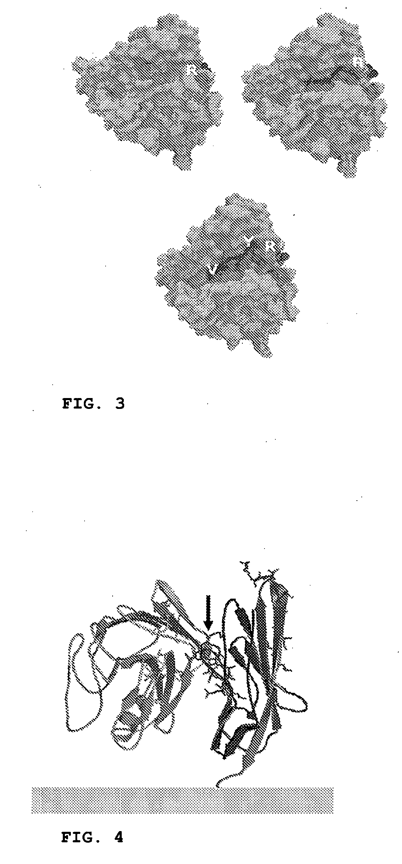

- la figure 3 est une représentation de la dynamique d'ouverture du domaine C-terminal de la protéine TSP-1 laissant apparaitre le peptide 4N1 (K-R-F-Y-V-V-M-W-K) dont les acides aminés sont représentés par des sphères de couleur plus foncée.
- la figure 4 illustre un modèle d'interaction moléculaire entre le domaine C-terminal de la TSP-1 (à gauche de la figure) et le domaine extracellulaire du récepteur CD47 (relié à la membrane cytoplasmique représentée en gris clair). Ce modèle est obtenu par la technique de docking protéine-protéine. La flèche visible entre les deux protéines correspond à la zone d'interaction moléculaire.

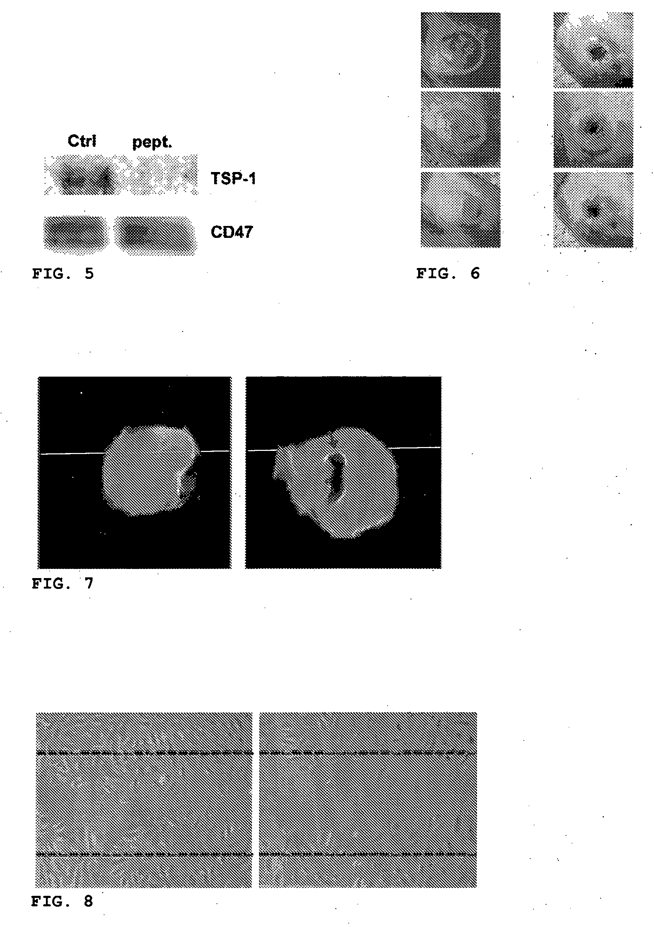

- la figure 5 représente l'inhibition de l'interaction biomoléculaire entre la TSP-1 et le récepteur CD47 de cellules MDA-MB-231 de cancer mammaire. Les cellules sont incubées pendant 2h en absence (Ctrl) ou en présence (pept.) de 100µM du peptide antagoniste selon l'invention. Les complexes TSP-1/CD47 sont alors immunoprécipités en utilisant un anticorps anti-CD47 puis la présence de TSP-1 et de CD47 est analysée grâce à la technique du Western-Blot.
- la figure 6 illustre l'action du peptide antagoniste cyclisé sur des cellules de mélanome murin. Après une injection sous-cutanée de 250 000 cellules B16F1 de mélanome murin à des souris syngéniques C57B1/6, des administrations intra-péritonéales de 10mg/kg du peptide antagoniste sont effectuées aux jours 3, 5 et 7. Les souris sont ensuite sacrifiées et les tumeurs sont photographiées au jour 20.
- la figure 7 correspond à une analyse IRM d'une tumeur non traitée (à gauche) et d'une tumeur traitée avec le peptide d'intérêt cyclisé au jour 12. Une flèche pointe sur la zone nécrotique.
- la figure 8 correspond à une photographie de cellules observées sous microscope à contraste de phase à un grossissement de 100X. Des cellules HUVEC (human umbilical vein endothelial cells) sont d'abord prétraitées avec une molécule anti-mitotique, la mitomycine (10µg/mL), pendant 2h à 37°C puis une blessure est réalisée sur le tapis cellulaire confluant. Les cellules sont alors incubées pendant 9h à 37°C avec le peptide décrit selon l'invention. La photographie de gauche correspond à des cellules « contrôle » tandis que celle de droite représente des cellules traitées avec le peptide d'intérêt (100uM).

Les protéines appartenant à la famille des thrombospondines, et en particulier les thrombospondines-1 et -2, respectivement TSP-1 et TSP-2, sont des macromolécules de la matrice extracellulaire. Elles interviennent dans la modulation de nombreuses interactions entre la matrice et la cellule et entre les cellules elles-mêmes.

Plus particulièrement, les thrombospondines sont des glycoprotéines présentant une structure en multi-domaines, chaque domaine étant impliqué dans diverses fonctions du fait de sa capacité à lier une multiplicité de récepteurs cellulaires de surface.

La famille des thrombospondines compte cinq protéines, TSP-1 à TSP-5, et est divisée en deux sous-groupes. Les TSP-1 et TSP-2 appartiennent au premier de ces deux groupes, du fait de leur structure commune en homotrimère consistant en trois sous-unités identiques. Ces trois monomères, présentant chacun une masse moléculaire approximative de 150 000 Da, sont reliés entre eux par des ponts disulfures. Les monomères de TSP-1 et de TSP-2 présentent un taux d'homologie qui va croissant de l'extrémité N-terminale (32% d'homologie) vers l'extrémité C-terminale (82% d'homologie). De plus, la plupart des séquences d'adhésion de la TSP-1 sont conservées dans la TSP-2. De ce fait, et comme rappelé ci-dessus, il est fort probable que la TSP-2 présente des fonctions similaires à la TSP-1.

Les TSP-3 à 5, quant à elles, sont des homopentamères. Leurs structures et leurs séquences sont donc différentes des TSP-1 et 2.

Comme nous l'avons vu précédemment, les thrombospondines sont des protéines retrouvées dans la matrice extracellulaire. Ainsi, ces protéines, et en particulier la TSP-1, influencent la structure et la composition de la matrice extracellulaire ainsi que le phénotype cellulaire du fait des interactions qu'elles peuvent établir.

*In vitro,* les cellules de la paroi vasculaire mises en culture, les cellules endothéliales, les cellules musculaires lisses et les fibroblastes, synthétisent et sécrètent la TSP-1 qu'elles incorporent également dans la matrice extracellulaire. *In vivo,* la TSP-1 est détectée dès les premiers stades de l'embryogenèse puis lors du développement embryonnaire dans les régions de migration cellulaire. Chez l'adulte, on la trouve notamment exprimée en concentration importante dans les tissus lésés ou inflammés au début de la réparation tissulaire. La TSP-1 peut également être synthétisée et sécrétée par des cellules impliquées dans la réponse immunitaire, telles que les plaquettes, les monocytes, les macrophages alvéolaires, etc.

Les thrombospondines, et en particulier la TSP-1, sont impliquées dans des processus cellulaires fondamentaux variés. Ainsi, la TSP-1 influence de nombreuses fonctions cellulaires, comme l'activation des plaquettes, l'angiogenèse, la cicatrisation, la mort cellulaire programmée et la progression tumorale (Sid et al., 2004). La TSP-2 présente une structure en domaines proche de celle de la TSP-1 et, de ce fait, certaines fonctions exercées par la TSP-2 sont similaires à celles de la TSP-1. En particulier, ces deux protéines inhibent l'angiogenèse (Mirochnik et al., 2008) qui est un processus permettant la croissance de nouveaux vaisseaux sanguins à partir de vaisseaux préexistants.

Plus particulièrement, des travaux de recherche menés par les inventeurs ont permis de mettre en évidence le rôle anti-apoptotique de la TSP-1 sur les cellules cancéreuses de carcinome thyroïdien humain (Rath et al., 2006). A ce titre, les inventeurs ont prouvé que la TSP-1 joue un rôle important dans la résistance des cellules tumorales aux agents chimiothérapeutiques, en particulier la doxorubicine et la camptothécine. Ces deux derniers exercent un effet cytotoxique en induisant l'apoptose chez de nombreux types cellulaires.

En se reportant à la figure 1, on remarque que l'activité caspase-3, considérée comme un bon marqueur des cellules entrant en apoptose, est considérablement inhibée en présence de TSP-1 par rapport à la même activité en présence de doxorubicine ou de camptothécine mais en absence de TSP-1. Cette dernière diminue donc le taux d'apoptose des cellules de carcinome thyroïdien soumises à un traitement anti-cancéreux.

Les auteurs ont également démontré que le niveau d'expression de la TSP-1 est corrélé au potentiel invasif des cellules de carcinome thyroïdien en utilisant deux lignées de cellules cancéreuses (FTC-138 et FTC-133) ayant une capacité d'invasion différente (Sid et al., 2008).

Par la suite, l'utilisation du peptide 4N1, présentant la séquence K-R-F-Y-V-V-M-W-K, a permis d'identifier l'extrémité C-terminale de la TSP-1 comme étant responsable de l'effet anti-apoptotique. En effet, ce peptide est capable d'établir une interaction moléculaire avec le récepteur CD47 ce qui va entrainer une réponse de la part de la cellule.

Le CD47, également appelé IAP pour « integrin-associated protein », est un récepteur cellulaire transmembranaire, appartenant à la famille des immunoglobulines, qui est exprimé sur une grande majorité des cellules. Le domaine extracellulaire du CD47 joue un rôle central dans la réponse des cellules et tissus humains après la liaison de la partie C-terminale de la TSP-1 ou de la TSP-2.

Les inventeurs ont donc cherché à comprendre le rôle du peptide 4N1 et du récepteur CD47 dans l'inhibition de l'apoptose des cellules cancéreuses, et en particulier des cellules FTC-133 de carcinome thyroïdien humain (Rath et al., 2006).

Comme on peut le voir sur la figure 2, en présence de peptide 4N1 exogène, la viabilité des cellules tumorales est plus importante par rapport à celle des cellules uniquement traitées avec l'une ou l'autre des deux drogues, camptothécine et doxorubicine, ou avec le peptide témoin 4NGG.

Le peptide 4N1, dérivé de l'extrémité C-terminale de la TSP-1, présente donc des propriétés anti-apoptotiques qui entrainent une résistance des cellules de carcinome thyroïdien aux molécules anti-cancéreuses de type camptothécine et doxorubicine. Au contraire, le peptide témoin 4NGG ne présente aucun effet sur la diminution du nombre de cellules tumorales induite par les deux médicaments précités.

De plus, en présence du peptide d'intérêt 4N1 et lorsqu'on ajoute l'anticorps B6H12 bloquant le CD47, ledit peptide ne permet plus la protection des cellules tumorales contre les anticancéreux utilisés.

Les différents résultats obtenus permettent donc de prouver que la thrombospondine, et en particulier la TSP-1, interagit avec les cellules tumorales en se liant par l'intermédiaire de son extrémité C-terminale à un récepteur, le CD47. Le récepteur CD47 représente donc une protéine membranaire « relai » dans le contrôle négatif de l'apoptose des cellules tumorales par le peptide 4N1 localisé au niveau de l'extrémité C-terminale de la TSP-1.

Ces résultats montrent également que de nombreuses séquences peptidiques proposées dans l'état de la technique, identiques ou très proches de celle du peptide 4N1, ne sont clairement pas adaptées pour une utilisation dans le cas présent. En effet, ledit peptide présente effectivement la capacité de se fixer sur le récepteur CD47 et, de ce fait, entraine une inhibition de la liaison entre ce dernier et une protéine de type TSP-1 ou TSP-2. Cependant, les résultats ci-dessus prouvent qu'une telle liaison entre le peptide 4N1 et le récepteur CD47 augmente la viabilité des cellules tumorales et leur résistance à l'encontre de certains médicaments. Le peptide 4N1 agit donc comme un agoniste du récepteur CD47.

C'est alors dans le cadre d'une démarche originale et inventive que les inventeurs ont cherché à approfondir les connaissances relatives aux interactions moléculaires, entre le récepteur CD47 et la TSP-1, responsables des effets biologiques cités précédemment, et en particulier l'effet anti-apoptotique sur les cellules tumorales.

La modélisation moléculaire de l'extrémité C-terminale de la TSP-1, et plus particulièrement la modélisation de l'interaction entre la région contenant la séquence 4N1 et le récepteur CD47, a donc été entreprise.

La technique d'analyse des modes normaux a ensuite été utilisée de manière à identifier les mouvements de la partie C-términale de la TSP-1 (Floquet et al., 2008). Cette technique a été plébiscitée car les seules simulations de dynamique moléculaire classiques et les méthodes biophysiques expérimentales ne permettent pas d'obtenir des résultats satisfaisants. De plus, l'interaction entre le récepteur CD47 et la TSP-1 ne peut pas être expliquée uniquement grâce à la structure cristallographique de la TSP-1 disponible dans la Protein Data Bank (PDB). En effet, la structure disponible de la TSP-1, obtenue par diffraction aux rayons-X, permet uniquement de décrire la séquence 4N1 comme étant complètement enfouie au sein d'une poche hydrophobe de la protéine TSP-1, rendant ainsi impossible toute interaction de ladite séquence avec un ligand.

Les résultats obtenus par Floquet et al. (2008), illustrés sur la figure 3, permettent d'expliquer le mécanisme aboutissant à l'interaction entre le peptide 4N1 et le récepteur CD47. En effet, les analyses ont permis notamment d'identifier que la poche hydrophobe, au niveau de la protéine TSP-1, s'ouvre lorsque celle-ci arrive à proximité du récepteur CD47 par l'intermédiaire d'un « scratch » électrostatique. Cette ouverture permet alors de révéler et de rendre accessible la séquence biologiquement active du peptide 4N1 ; l'interaction entre la TSP-1, par l'intermédiaire dudit peptide, et le CD47 peut alors s'effectuer.

Le mouvement d'ouverture de la poche hydrophobe a alors été exploré plus en détails et cela a permis de générer différentes structures plus ou moins ouvertes de la protéine TSP-1.

En parallèle, plusieurs modèles de la partie extracellulaire du récepteur CD47 ont été générés par la technique de modélisation par homologie.

Des analyses supplémentaires, effectuées grâce à la méthode de docking protéine-protéine, ont également permis de prédire la région d'interaction potentielle entre la structure ouverte de la TSP-1 et le récepteur CD47. Les résultats obtenus sont visibles sur la figure 4.

Les modèles d'interaction obtenus ont alors permis de proposer des fragments peptidiques mimant les séquences du récepteur CD47 impliquées dans l'interaction avec la protéine TSP-1.

Les peptides proposés permettent donc avantageusement de se fixer au niveau de l'extrémité C-terminale de la TSP-1, et en particulier au niveau de la région d'interaction constituée par le peptide 4N1, lorsque celui-ci est accessible, notamment dans le cas de la proximité du récepteur membranaire CD47. En d'autres termes, les peptides selon l'invention permettent d'antagoniser l'interaction TSP-1/CD47 en se fixant très spécifiquement sur la TSP-1 au niveau du site de liaison entre ces deux protéines.

De cette manière, le récepteur cellulaire CD47 reste libre et peut interagir librement avec ses ligands habituels, outre la TSP-1. De plus, les autres domaines de la protéine TSP-1 conservent également leur capacité de liaison. Le peptide selon la présente invention est donc particulièrement avantageux lorsqu'on connait l'importance des deux protéines TSP-1 et CD47, dans de nombreux processus cellulaires fondamentaux.

Selon un mode de réalisation particulièrement intéressant, le peptide retenu pour antagoniser la liaison entre la protéine TSP-1 et le récepteur CD47 correspond à une courte séquence du domaine extracellulaire dudit récepteur, et plus particulièrement en un dodécapeptide présentant la séquence S1 suivante :
S1 : R1-R2-R3-S-Q-L-L-K-G-R4-R5-R6

Préférentiellement, R1 correspond à un acide aminé appartenant au groupe des acides aminés apolaires, c'est-à-dire l'isoleucine (I) ou la leucine (L) ou la valine (V) ou l'alanine (A). Il en est de même pour R3 et R5

Selon un mode de réalisation intéressant, l'acide aminé R2 appartient au groupe des acides aminés polaires et chargés négativement, c'est-à-dire l'acide glutamique (E) ou l'acide aspartique (D). Il en est de même pour R4

Avantageusement, l'acide aminé R6 appartient au groupe des acides aminés polaires et non chargés présentant un radical hydroxyle (-OH). Ainsi R6 correspond soit à la sérine (S), soit à la thréonine (T).

L'hexapeptide central, de formule S-Q-L-L-K-G, est particulièrement avantageux pour permettre la liaison du peptide selon l'invention au niveau du domaine C-terminal de la TSP-1. Les acides aminés qui le précèdent ou le suivent dans la séquence S1 peuvent être modifiés mais il est préférable de conserver cet hexapeptide pour une liaison optimale avec la protéine TSP-1.

Selon un mode de réalisation particulièrement avantageux, le dodécapeptide selon la présente invention présente une séquence S1 qui correspond à la séquence identifiée S2 suivante :
S2: I-E-V-S-Q-L-L-K-G-D-A-S

En effet, cette séquence S2 particulière est optimale pour antagoniser l'interaction de la protéine TSP-1 avec le récepteur CD47.

On sait déjà, de part les résultats des recherches antérieures menées par les inventeurs, que le fait de bloquer la liaison TSP-1/CD47 entraine une levée de l'inhibition de l'apoptose des cellules cancéreuses de carcinome thyroïdien humain (Rath et al., 2006). De ce fait, l'utilisation du peptide selon l'invention permet, en particulier mais non limitativement, une inhibition de l'invasion des tissus par les cellules tumorales de carcinome thyroïdien.

Des travaux ont alors ont été conduits pour confirmer ces résultats en utilisant des cellules tumorales provenant d'autres types de cancer.

Ainsi, des cellules MDA-MB-231, correspondant à des cellules provenant d'un cancer mammaire, ont été incubées pendant 24h, avec ou sans le dodécapeptide selon la présente invention. En particulier, le peptide qui a été utilisé est celui qui présente la séquence S2 : I-E-V-S-Q-L-L-K-G-D-A-S.

Une immunoprécipitation des complexes, avec un anticorps dirigé contre le CD47, puis une analyse des protéines TSP-1 et CD47 par Western-Blot ont ensuite été effectuées. Les résultats sont illustrés sur la figure 5. Comme on peut le voire, la TSP-1 n'est plus détectée par l'analyse Western-Blot après l'immunoprécipitation lorsque les cellules cancéreuses ont été incubées en présence du peptide antagoniste selon l'invention. Cela signifie que le traitement réalisé avec ledit peptide permet d'abolir l'interaction moléculaire entre la protéine TSP-1 et le récepteur CD47.

L'invention concerne également un polypeptide comportant un nombre inférieur à 50 acides aminés et incorporant un peptide présentant un pourcentage d'homologie d'au moins 60% avec la séquence S2 selon l'invention, de préférence 80% d'homologie et de préférence encore 95% d'homologie, ledit peptide comprenant un hexapeptide central de séquence S-G-L-L-K-G.

En effet, une proportion de 7 acides aminés sur un total de 12, ce qui correspond à un pourcentage d'homologie de 60%, constitue une séquence minimale active suffisante pour inhiber la liaison entre la TSP-1 et les récepteur CD47.

L'utilisation d'analogues structuraux non peptidiques de la séquence selon l'invention est également envisageable pour inhiber la liaison TSP-1/CD47.

Selon un exemple de réalisation particulièrement intéressant, le peptide selon l'invention est cyclisé. En effet, il s'avère que le segment de CD47 correspondant au.peptide de la présente invention forme une boucle au sein du récepteur. Ainsi, une telle cyclisation est avantageuse car elle permet de stabiliser l'interaction entre ledit peptide et l'extrémité C-terminale de la protéine TSP-1, ce qui a pour effet d'améliorer l'activité biologique et l'efficacité du peptide. Par ailleurs, des calculs de dynamique moléculaire sur différents peptides dérivés ont montré qu'il était possible de stabiliser la structure locale en opérant une cyclisation desdits peptides.

La cyclisation du peptide antagoniste selon l'invention peut être effectuée par tout moyen apte à cet effet et connu de l'homme du métier. En particulier, il est avantageux de procéder à la cyclisation par l'intermédiaire d'une liaison amidique.

Plus préférentiellement encore, la cyclisation du peptide d'intérêt est opérée par l'intermédiaire d'un pont disulfure, qui est un lien covalent fort qui réunit les fonctions thiols (-SH) de deux acides aminés de type cystéine (C).

Le choix du pont disulfure pour obtenir un cyclopeptide est particulièrement avantageux car il permet de conserver ledit peptide sous forme zwitterion à un pH physiologique. Une molécule présentant une forme de zwitterion comprend une charge positive au niveau du groupement amine et une charge négative au niveau du groupement carboxyle. La forme zwitterion est avantageuse car elle permet à la molécule de conserver une bonne solubilité dans un solvant aqueux.

Ainsi, selon un exemple de réalisation particulièrement préféré de l'invention, le dodécapeptide présente une séquence S1 qui correspond à la séquence identifiée S3 : C-E-V-S-Q-L-L-K-G-D-A-C. Les radicaux R1 et R6 présents aux deux extrémités ont été remplacés chacun par une cystéine. Les deux acides aminés cystéine (C) aux extrémités du peptide vont permettre la formation d'un pont disulfure (-S-S-) entre leurs fonctions thiols respectives.

Le peptide présentant la séquence S3 ci-dessus présente les mêmes résultats que le peptide non cyclisé de séquence S2 en ce qui concerne l'expérience d'immunoprécipitation des complexes TSP-1/CD47 réalisée en présence d'anticorps anti-CD47. En conclusion, le cyclopeptide permet également d'abolir l'interaction moléculaire entre la protéine TSP-1 et le récepteur CD47.

L'action du dodécapeptide cyclisé de séquence S3 C-E-V-S-Q-L-L-K-G-D-A-C a également été testée directement in *vivo* sur un modèle de mélanome murin B16F1 injecté à des souris syngéniques C57B1/6. Les résultats sont représentés sur les figures 6 et 7.

La figure 6 illustre l'aspect des tumeurs au jour 20 suivant l'injection des cellules de mélanome aux souris C57B1/6 et après traitement éventuel de l'animal avec le peptide d'intérêt cyclisé. La moitié des souris (4/8) qui ont été traitées avec ledit peptide (à droite sur la figure) montrent une forte zone nécrotique localisée au niveau de la tumeur tandis qu'aucune des souris « contrôle » ne présente une telle nécrose.

La figure 7 permet de comparer, par une analyse d'imagerie par résonance magnétique (IRM), la tumeur prélevée sur l'animal non traité avec la tumeur prélevée sur l'animal traité par le peptide cyclisé par l'intermédiaire d'un pont disulfure au jour 12 après l'injection des cellules tumorales.

On remarque sur cette figure que la tumeur issue de l'animal traité à l'aide du peptide cyclisé selon l'invention présente une zone nécrotique qui est mise en évidence à l'aide d'une flèche. Au contraire, la même tumeur issue d'un animal non traité ne présente aucune zone nécrotique. Ledit peptide présente donc une activité antitumorale.

Le peptide selon la présente invention, et notamment le peptide cyclisé, peut donc être utilisé dans le traitement du cancer folliculaire thyroïdien, du cancer mammaire ou du mélanome. Cependant, de telles utilisations ne sont pas limitatives et on peut aisément imaginer que le peptide selon la présente invention soit utilisé pour le traitement de nombreux cancers différents de ceux cités ci-dessus.

Un autre avantage de l'invention, illustré figure 8, est que le peptide d'intérêt permet d'inhiber l'angiogenèse tumorale.

L'angiogenèse est un processus physiologique normal, qui intervient par exemple lors de la croissance embryonnaire. Cependant, l'angiogenèse peut également correspondre à un processus pathologique primordial dans la croissance des tumeurs cancéreuses malignes et dans la prolifération des métastases. En effet, les cellules malignes ont besoin d'oxygène et de nutriments pour leur croissance. Pour cela, les cellules vont induire la formation de nouveaux vaisseaux sanguins à partir de vaisseaux préexistants au niveau de tissus sains adjacents. Lorsque ces nouveaux vaisseaux sont formés, ils permettent d'une part de faciliter la croissance de la tumeur mais également la dissémination des cellules cancéreuses vers des organes distants.

L'angiogenèse est un processus se déroulant en trois phases :
- le *sprouting* (bourgeonnement) qui correspond dans un premier temps à une activation des cellules entrainant une dégradation de la membrane basale et de la matrice extracellulaire environnante puis à une migration des cellules endothéliales. Les cellules prolifèrent et se différencient en une structure de type capillaire pour former un vaisseau sanguin.
- l'*intussusception* au cours de laquelle un élargissement et une séparation des vaisseaux sanguins déjà formés va s'opérer.
- la *septation* pendant laquelle les cellules endothéliales poussent à l'intérieur des vaisseaux créant des canaux vasculaires séparés.

Les thrombospondines, et en particulier la TSP-1, sont connues pour jouer un rôle dans la modulation de l'angiogenèse, notamment dans le contexte tumoral.

L'interaction entre le récepteur CD47 et la TSP-1 en particulier est connue pour présenter un effet anti-angiogénique. L'utilisation, dans le contexte tumoral, du peptide selon l'invention pouvait donc s'avérer a *priori* risquée du fait de la suppression de cet effet anti-angiogénique et du risque d'une potentielle vascularisation de la tumeur. Cependant, les études menées par les inventeurs montrent que, contrairement à ce qui pouvait être attendu et de façon très surprenante, le peptide selon l'invention présente un effet anti-angiogénique sur les cellules testées.

En effet, la TSP-1, outre son interaction avec le récepteur CD47, peut également se lier au récepteur CD36. Une telle interaction TSP-1/CD36 aurait donc une action anti-angiogénique sur les cellules tumorales et/ou endothéliales. Grâce au peptide selon la présente invention, et comme nous l'avons vu précédemment, l'interaction TSP-1/CD47 est abolie. Cependant, la liaison entre la TSP-1 et le récepteur CD36 peut toujours avoir lieu, du fait de l'extrême spécificité du peptide d'intérêt qui n'empêche pas la TSP-1 de se lier avec d'autres ligands. De plus, étant donné que la TSP-1 ne se lie plus au récepteur CD47, une proportion plus importante de TSP-1 va pouvoir se fixer au CD36 ce qui va entrainer une inhibition d'autant plus importante de l'angiogenèse tumorale.

La photographie de la figure 8 illustre la migration de cellules HUVEC, en présence et en absence de peptide antagoniste, après que ces cellules aient été soumises à une blessure dont les contours sont représentés en traits pointillés. Les résultats sont représentatifs de 4 expériences indépendantes et ils montrent clairement que la première phase du processus d'angiogenèse, à savoir la migration des cellules, est inhibée en présence du peptide antagoniste selon l'invention.

Ainsi, du fait de l'extrême spécificité du peptide antagoniste selon l'invention, la protéine TSP-1 peut toujours se lier au récepteur CD36. Cependant, d'autres interactions entre la TSP-1 et/ou le CD47 et des protéines membranaires, ou mêmes solubles sont également possibles. En particulier, des interactions avec des protéines membranaire de type intégrine, HSPG (heparin sulfate proteoglycan), SIRP (signal regulatory protein) etc. pourraient éventuellement participer et même renforcer l'effet antitumoral et/ou l'effet anti-angiogénique du peptide antagoniste.

En ce qui concerne la synthèse du peptide d'intérêt selon la présente invention, celle-ci peut être effectuée, en particulier mais non limitativement, par voie recombinante. A cet effet, une séquence d'acide nucléique, préférentiellement de l'ADN, codant pour ledit peptide peut être introduit dans une cellule hôte, préférentiellement par l'intermédiaire d'un vecteur.

Ainsi la présente invention concerne également un acide nucléique isolé codant pour le peptide selon l'invention. Préférentiellement, il s'agit d'ADN mais il peut également s'agir d'ARN.

Plus préférentiellement encore, ledit peptide peut également être acquis par une technique de synthèse chimique peptidique classique connue de l'homme du métier.

Le peptide selon la présente invention comporte donc des avantages nombreux et variés. Par exemple, sa courte séquence est facile à obtenir, notamment par les techniques classiques de synthèse chimique.

Cependant, l'avantage le plus considérable réside dans le fait que le peptide bloque spécifiquement la liaison TSP-1/CD47. Plus particulièrement, il s'agit de l'extrémité C-terminale de la TSP-1 qui est bloquée. Ainsi, les autres domaines de la protéine TSP-1 peuvent continuer à interagir avec leurs ligands respectifs. En particulier, le domaine de la TSP-1 comprenant le domaine de répétition de type I (domaine d'homologie à la properdine) peut interagir avec le récepteur cellulaire CD36, ce qui entraine une inhibition de l'angiogenèse. Ainsi, on obtient une diminution de la vascularisation de la tumeur et, de ce fait, une inhibition de l'invasion par les cellules cancéreuses. Le récepteur CD47 reste également libre et peut alors lier ses ligands naturels. De plus, il n'y a aucun risque que le peptide selon l'invention soit un agoniste du récepteur CD47, comme peuvent l'être des molécules déjà utilisées dans l'état de la technique pour bloquer l'interaction TSP-1/CD47, telles que les anticorps anti-CD47 ou le peptide 4N1.

Ainsi, le peptide antagoniste présente de nombreuses applications potentielles dans des pathologies variées, notamment en pathologie tumorale, et particulièrement dans le carcinome folliculaire thyroïdien, le cancer mammaire ou le mélanome, où il devrait permettre de limiter le développement tumoral et/ou métastatique des cellules cancéreuses. Par exemple, ledit peptide permettrait une potentialisation des effets des traitements chimiothérapeutiques qui induisent l'apoptose chez certains types de cellules cancéreuses ; en effet, le peptide d'intérêt contribuerait à diminuer la résistance des cellules cancéreuses à ces traitements.

Enfin, étant donné que de nombreuses références bibliographiques attribuent à la protéine TSP un fort potentiel vasoconstricteur et que le peptide, selon l'invention, est susceptible d'avoir un impact sur le développement de stratégies pro-angiogéniques, des applications potentielles dans le domaine des pathologies cardiovasculaires et cérébrales peuvent donc être envisagées.

### REFERENCES BIBLIOGRAPHIQUES

- N. Esemuede, T. Lee, D. Pierre-Paul, B. Sumpio, V. Gahtan, The role of thrombospondin-1 in human disease. J. Surg. Res. 122 (2004) 135-142
- N. Floquet, S. Dedieu, L. Martiny, M. Dauchez, D. Perahia, Human thrombospondin's (TSP-1) C-terminal domains open to interact with the CD-47 receptor: a molecular modeling study. Arch. Biochem. Biophys. 478 (2008) 103-109
- P. Manna & W. Frazier, CD47 mediates killing of breast tumor cells via Gi-dependent inhibition of protein kinase A. Cancer Res.64(2004) 1026-36
- Y. Mirochnik, A. Kwiatek, O. Volpert, Thrombospondin and apoptosis: molecular mechanisms and use for design of complementation treatments Curr. Drug Targets 2008 9(10) 851-862
- G. Rath, C. Schneider, S. Dedieu, B. Rothhut, M. Soula-Rothhut, C. Ghoneim, B. Sid, H. Morjani, H. El Btaouri, L. Martiny, The C-terminal CD47/IAP-bindig domain of thrombospondin-1 prevents camptothecin- and doxorubicin-induced apoptosis in human thyroid carcinoma cells. Biochim. Biophys. Acta 1763 (2006) 1125-1134
- B. Sid, B. Langlois, H. Sartelet, G. Bellon, S. Dedieu, L. Martiny, Thrombospondin-1 enhances human thyroid carcinoma cell invasion through urokinase activity. Int. J. Biochem. Cell. Bio. 40 (2008) 1890-1900
- B. Sid, H. Sartelet, G. Bellon, H. El Btaouri, G. Rath, N. Delorme, B. Haye, L. Martiny, Thrombospondin 1: a multifunctional protein implicated in the regulation of tumor growth. Crit. Rev. Oncol. Hematol. 49 (2004) 245-258
- T.Wang, X. Qian, M. Granick, M. Solomon, V. Rothman, D. Berger & Tuszynski, Inhibition of breast cancer progression by an antibody through a thrombospondin-1 receptor. Surgery 120 (1996a) 449-454
- T.Wang, X. Qian, M. Granick, M. Solomon, V. Rothman, D. Berger & Tuszynski, Thrombospondin-1 (TSP-1) promotes the invasive properties of human breast cancer. J. Surg. Res. 63(1996b) 39-43

## Revendications

1. Peptide antagoniste de la liaison entre un récepteur CD47, et une protéine appartenant à la famille des thrombospondines, ou TSP, **caractérisé en ce qu'**il présente la séquence S1 suivante :
S1: R1-R2-R3-S-Q-L-L-K-G-R4-R5-R6
où R1 à R6 correspondent à des acides aminés.

2. Peptide selon la revendication 1, **caractérisé en ce que** R1, R3 et R5 correspondent chacun à un acide aminé apolaire choisi parmi l'isoleucine (I) et/ou la leucine (L) et/ou la valine (V) et/ou l'alanine (A).

3. Peptide selon l'une quelconque des revendications précédentes, **caractérisé en ce que** R2 et R4 correspondent chacun à un acide aminé polaire chargé négativement choisi parmi l'acide glutamique (E) et/ou l'acide aspartique (D).

4. Peptide selon l'une quelconque des revendications précédentes, **caractérisé en ce que** R6 correspond à un acide aminé, polaire et non chargé comportant un radical hydroxyle choisi parmi la sérine (S) ou la thréonine (T).

5. Peptide selon l'une quelconque des revendications précédentes, **caractérisé en ce que** la séquence S1 dudit peptide est égale à la séquence suivante identifiée séquence S2 :
S2: I-E-V-S-Q-L-L-K-G-D-A-S

6. Peptide selon l'une quelconque des revendications précédentes, **caractérisé en ce que** ledit peptide est cyclisé.

7. Peptide selon la revendication 1, **caractérisé en ce qu'** il est cyclisé par l'intermédiaire d'un pont disulfure entre deux acides aminés de type cystéine (C) et **en ce que** ledit peptide présente la séquence S1 suivante, identifiée séquence S3:
S3: C-E-V-S-Q-L-L-K-G-D-A-C

8. Peptide selon l'une quelconque des revendications précédentes, **caractérisé en ce qu'**il est obtenu par voie recombinante.

9. Peptide selon l'une quelconque des revendications 1 à 7, **caractérisé en ce qu'**il est obtenu par synthèse chimique.

10. Peptide selon l'une quelconque des revendications précédentes pour le traitement du cancer.

11. Peptide selon la revendication 10 pour le traitement du cancer folliculaire thyroïdien.

12. Peptide selon la revendication 10 pour le traitement du cancer mammaire.

13. Peptide selon la revendication 10 pour le traitement du mélanome.

14. Peptide l'une quelconque des revendications 10 à 13 pour empêcher l'interaction entre la thrombospondine-1 et le récepteur CD47 et inhiber l'effet anti-apoptotique de cette interaction sur les cellules cancéreuses.

15. Peptide selon l'une quelconque des revendications 10 à 13 pour empêcher l'interaction entre la thrombospondine-2 et le récepteur CD47 et inhiber l'effet anti-apoptotique de cette interaction sur les cellules cancéreuses.

16. Acide nucléique isolé codant pour un peptide selon l'une quelconque des revendications 1 à 8.

## Patentansprüche

1. Peptidantagonist der Bindung zwischen einem CD47-Rezeptor und einem zu der Thrombospondine-Familie gehörendem Protein, oder TSP, **dadurch gekennzeichnet, dass** er die folgende S1 Sequenz aufweist:
S1: R1-R2-R3-S-Q-L-L-K-G-R4-R5-R6
wo R1 bis R6 Aminosäuren entsprechen.

2. Peptid nach Anspruch 1, **dadurch gekennzeichnet, dass** R1, R3 und R5 jeweils einer apolaren Aminosäure entsprechen, die aus Isoleucin (I) und/oder Leucin (L) und/oder Valin (V) und/oder Alanin (A) gewählt ist.

3. Peptid nach einem der vorhergehenden Ansprüche, **dadurch gekennzeichnet, dass** R2 und R4 jeweils einer negativ geladenen polaren Aminosäure entsprechen, die aus Glutaminsäure (E) und/oder Asparaginsäure (D) gewählt ist.

4. Peptid nach einem der vorhergehenden Ansprüche, **dadurch gekennzeichnet, dass** R6 einer polaren und ungeladenen Aminosäure entspricht, die einen aus Serin (S) oder Threonin (T) gewählten Hydroxylrest umfasst.

5. Peptid nach einem der vorhergehenden Ansprüche, **dadurch gekennzeichnet, dass** die S1 Sequenz des besagten Peptids gleich der folgenden Sequenz ist, die als S2 Sequenz identifiziert wird:
S2: I-E-V-S-Q-L-L-K-G-D-A-S

6. Peptid nach einem der vorhergehenden Ansprüche, **dadurch gekennzeichnet, dass** das Peptid cyclisiert ist.

7. Peptid nach Anspruch 1, **dadurch gekennzeichnet, dass** es über eine DisulfidBrücke zwischen zwei Aminosäuren der Art Cystein (C) cyclisiert ist und dass das besagte Peptid die folgende S1 Sequenz aufweist, die als S3 Sequenz identifiziert wird:
S3: C-E-V-S-Q-L-L-K-G-D-A-C

8. Peptid nach einem der vorhergehenden Ansprüche, **dadurch gekennzeichnet, dass** es rekombinant erhalten wird.

9. Peptid nach einem der Ansprüche 1 bis 7, **dadurch gekennzeichnet, dass** es durch chemische Synthese erhalten wird.

10. Peptid nach einem der vorhergehenden Ansprüche zur Behandlung von Krebs.

11. Peptid nach Anspruch 10 zur Behandlung von follikulären Schilddrüsenkrebs.

12. Peptid nach Anspruch 10 zur Behandlung von Brustkrebs.

13. Peptid nach Anspruch 10 zur Behandlung von Melanomen.

14. Peptid nach einem der Ansprüche 10 bis 13 zum Verhindern der Interaktion zwischen Thrombospondin-1 und dem CD47-Rezeptor und zum Hemmen der anti-apoptotischen Wirkung dieser Interaktion auf die Krebszellen.

15. Peptid nach einem der Ansprüche 10 bis 13 zum Verhindern der Interaktion zwischen Thrombospondin-2 und dem CD47-Rezeptor und zum Hemmen der anti-apoptotischen Wirkung dieser Interaktion auf die Krebszellen.

16. Isolierte Nukleinsäure, die ein Peptid nach einem der Ansprüche 1 bis 8 kodiert.

## Claims

1. A peptide antagonist of the binding between a CD47 receptor and a protein belonging to the thrombospondin family, or TSP, wherein it has the following S1 sequence:
S1 : R1-R2-R3-S-Q-L-L-K-G-R4-R5-R6
where R1 to R6 correspond to amino acids.

2. The peptide according to claim 1, wherein R1, R3 and R5 each correspond to an apolar amino acid selected from isoleucine (I) and/or leucine (L) and/or valine (V) and/or alanine (A).

3. The peptide according to any one of the preceding claims, wherein R2 and R4 each correspond to a negatively charged polar amino acid selected from glutamic acid (E) and/or aspartic acid (D).

4. The peptide according to any one of the preceding claims, wherein R6 corresponds to a polar and uncharged amino acid including a hydroxyl radical selected from serine (S) or threonine (T).

5. The peptide according to any one of the preceding claims, wherein the S1 sequence of said peptide is equal to the following sequence identified as S2 sequence:
S2: I-E-V-S-Q-L-L-K-G-D-A-S

6. The peptide according to any one of the preceding claims, wherein said peptide is cyclized.

7. The peptide according to claim 1, wherein it is cyclized through a disulfide bridge between two amino acids of the cysteine type (C) and said peptide has the following S1 sequence, identified as S3 sequence:
S3: C-E-V-S-Q-L-L-K-G-D-A-C

8. The peptide according to any one of the preceding claims, wherein it is obtained recombinantly.

9. The peptide according to any one of claims 1 to 7, wherein it is obtained by chemical synthesis.

10. The peptide according to any preceding claim for the treatment of cancer.

11. The peptide according to claim 10 for the treatment of thyroid follicular cancer.

12. The peptide according to claim 10 for the treatment of breast cancer.

13. The peptide according to claim 10 for the treatment of melanoma.

14. The peptide according to any one of claims 10 to 13 for preventing the interaction between thrombospondin-1 and the CD47 receptor and inhibiting the anti-apoptotic effect of this interaction on the cancerous cells.

15. The peptide according to any one of claims 10 to 13 for preventing the interaction between thrombospondin-2 and the CD47 receptor and inhibiting the anti-apoptotic effect of this interaction on the cancerous cells.

16. An isolated nucleic acid encoding a peptide according to any one of claims 1 to 8.
